(19) Europäisches Patentamt
European Patent Office
Office européen des brevets

(11) EP 3 562 802 B1

(12) EUROPEAN PATENT SPECIFICATION

(45) Date of publication and mention of the grant of the patent:
**29.09.2021 Bulletin 2021/39**

(21) Application number: **16925543.7**

(22) Date of filing: **30.12.2016**

(51) Int Cl.:
***C07C 37/20*** *(2006.01)* ***B01J 31/10*** *(2006.01)*
***C07C 39/16*** *(2006.01)*

(86) International application number:
**PCT/CN2016/113515**

(87) International publication number:
**WO 2018/120024 (05.07.2018 Gazette 2018/27)**

# (54) RESIN WITH PROMOTER AND ANTIOXIDANT

HARZ MIT PROMOTOR UND ANTIOXIDANS

RÉSINE AVEC PROMOTEUR ET ANTIOXYDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.11.2019 Bulletin 2019/45**

(73) Proprietors:
- **DDP Specialty Electronic Materials US, LLC Wilmington, DE 19805 (US)**
- **DDP Specialty Electronic Materials US 8, LLC Wilmington, DE 19805 (US)**

(72) Inventors:
- **COSTEUX, Chunxia Midland Michigan 48674 (US)**
- **ZU, Chengli Midland Michigan 48667 (US)**
- **YANG, Liu Shanghai 201203 (CN)**
- **SCHULTZ, Alfred K. Collegeville Pennsylvania 19426 (US)**

(74) Representative: **Houghton, Mark Phillip Patent Outsourcing Limited Cornerhouse 1 King Street Bakewell Derbyshire DE45 1DZ (GB)**

(56) References cited:
**CN-A- 1 479 652**
**CN-A- 1 524 181**
**CN-A- 101 111 314**
**CN-A- 103 974 925**
**CN-A- 103 987 459**
**US-A- 4 595 704**
**US-A- 4 973 607**
**US-A- 4 973 607**
**US-A1- 2014 378 715**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 562 802 B1

## Description

Resin beads that have sulfonic acid groups are useful for a variety of purposes. For example, such resin beads are used as catalysts for useful chemical reactions such as the condensation of phenol with acetone to produce bisphenol-A. When resin beads are used as catalysts, it is sometimes useful to mix the resin beads with one or more promoter, which may not be chemically bound to the resin beads. The presence of the promoter improves the action of the catalyst in some way, for example by improving the selectivity of the reaction that is conducted in the presence of the catalyst. It is also desirable to store resin beads prior to using them as catalysts, and it is desired that little or no degradation of the resin beads takes place during the storage. One method that has been used to prevent degradation of the resin beads during storage is to mix the resin beads with one or more antioxidant, which may or may not be chemically bound to the resin beads.

US 8,907,138 describes use of an antioxidant treated promoted strong acid ion exchange resin as an acid catalyst. The acid catalyst described in US 8,907,138 was not subjected to long-term storage or to elevated temperature during storage.

US 2014/0378715 discloses the use of an antioxidant treated promoted strong acid ion exchange resin as an acid catalyst.

In the course of developing the present invention, it has been discovered that cysteamine, a frequently-used thiol-amine promoter, reacts chemically with a frequently-used antioxidant. Sometime, cysteamine is added directly to a collection of resin beads, and sometimes 2,2-dimethylthiazolidine ("DMT") is added to a collection resin beads, because it is considered that DMT can (for example, when water is present) undergo a chemical reaction to produce cysteamine. It has been discovered that, when cysteamine and that antioxidant are both present in a collection of resin beads, a chemical reaction takes place that destroys some or all of the antioxidant, thus leaving the resin beads vulnerable to degradation, especially during long-term and/or heated storage. It is desired to provide an antioxidant that does not react with thiol-amine promoter during long-term and/or heated storage.

The present invention in all its aspects is set out in the accompanying claims.

Accordingly, the invention provides is a catalyst composition comprising

(a) a collection of resin beads having sulfonic acid functional groups;
(b) one or more promoter selected from structures (III) and (IV)

$$H_2N-CH_2-R^{15}-CH_2-SH$$

(III)

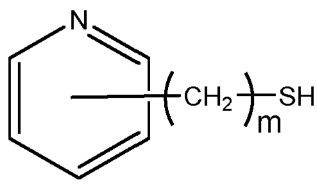

(IV)

where $R^{15}$ is $(CH_2)_p$ where p is 0 to 10, and m is 1 to 10; or 2,2-dimethylthiazolidine; and
(c) an antioxidant having the structure (I)

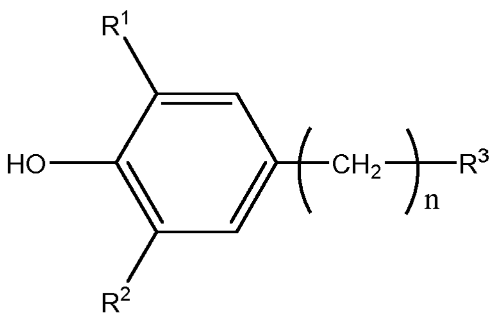

(I)

wherein each of $R^1$ and $R^2$ is an unsubstituted alkyl group containing 3 to 12 carbon atoms;
wherein $R^3$ is hydrogen or methyl and n is 0 to 20.

The following is a brief description of the drawings.

Figures 1-6 show the results of LC/UV/MS analysis of solutions, with the solutes as follows. Fig. 1 was ETHANOX™ 703. Figure 2 was ETHANOX™ 703 and DMT. Figure 3 was DtBP, and Figure 4 was DtBP and DMT. Figure 5 was BHT, and Figure 6 was BHT and DMT.

The following is a detailed description of the invention.

As used herein, the following terms have the designated definitions, unless the context clearly indicates otherwise.

As used herein, "resin" is synonymous with "polymer." "Polymer," as used herein, refers to relatively large molecules made up of the reaction products of smaller chemical repeat units. Polymers may have structures that are linear, branched, star shaped, looped, hyperbranched, crosslinked, or a combination thereof; polymers may have a single type of repeat unit ("homopolymers") or they may have more than one type of repeat unit ("copolymers"). Copolymers may have the various types of repeat units arranged randomly, in sequence, in blocks, in other arrangements, or in any mixture or combination thereof. A polymer has weight-average molecular weight of 2,000 or higher. A polymer that is sufficiently crosslinked that is insoluble in all solvents is considered to have infinite molecular weight.

Vinyl monomers have the structure (II)

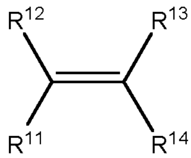

(II)

where each of $R^{11}$, $R^{12}$, $R^{13}$, and $R^{14}$ is, independently, a hydrogen, a halogen, an aliphatic group (such as, for example, an alkyl group), a substituted aliphatic group, an aryl group, a substituted aryl group, another substituted or unsubstituted organic group, or any combination thereof. Vinyl monomers are capable of free radical polymerization to form polymers. Some vinyl monomers have one or more polymerizable carbon-carbon double bonds incorporated into one or more of $R^{11}$, $R^{12}$, $R^{13}$, and $R^{14}$, and such vinyl monomers are known herein as multifunctional vinyl monomers. Vinyl monomers with exactly one polymerizable carbon-carbon double bond are known herein as monofunctional vinyl monomers.

Styrenic monomers are vinyl monomers in which each of $R^{11}$, $R^{12}$, and $R^{13}$ is hydrogen or an unsubstituted alkyl and $R^{14}$ contains one or more substituted or unsubstituted aromatic ring.

Acrylic monomers are vinyl monomers in which each of $R^{11}$ and $R^{12}$ is hydrogen; $R^{13}$ is either hydrogen or methyl; and $R^{14}$ is a carboxyl group, a carboxylate ester group (in which the ester group is a substituted or unsubstituted alkyl group), an amide group (in which the nitrogen atom is bonded to zero, one, or two substituted or unsubstituted alkyl groups).

A reaction among monomers to form one or more polymers is referred to herein as a polymerization process. The residue of a monomer after a polymerization process has taken place is known herein as a polymerized unit of that monomer.

Polymer beads are individual particles, each containing 50% or more by weight of polymer. Beads are in the solid state at 23°C. Beads have volume-average diameter of 10 $\mu$m or greater. If a particle is not spherical, the diameter of the particle is taken herein to be the diameter of an imaginary sphere that has the same volume as the particle.

As used herein, alkyl groups and alkenyl groups may be linear, branched, cyclic, or a combination thereof.

As used herein, a chemical group is said herein to be "substituted" if a substituent (that is, an atom or chemical group) is attached. Suitable substituents include, for example, alkyl groups, alkynyl groups, aryl groups, halogen atoms, nitrogen-containing groups including amine groups (including mono- and dialkylamine groups), oxygen-containing groups (including carboxyl groups and oxyalkyl groups), sulfur-containing groups (including sulfonic acid groups), nitrile groups, and combinations thereof.

As used herein, a heterocyclic aromatic group is a group that contains an aromatic ring in which one or more member of the aromatic ring is a heteroatom. A heteroatom is an atom other than carbon and hydrogen.

A collection of particles is characterized by the volume-based distribution of diameters. The parameter D60 denotes the value of a diameter that has the property that exactly 60% of the collection of the particles, by volume, have diameter less than or equal to D60. The parameter D10 denotes the value of a diameter that has the property that exactly 10% of the collection of the particles, by volume, have diameter less than or equal to D10. The parameter "uniformity coefficient" (abbreviated "UC") is UC = D60 / D10.

A collection of particles is also characterized by the harmonic mean diameter (HMD), which is defined as

$$HMD = \frac{N}{\sum_{i=1}^{N}\left(\frac{1}{d_i}\right)}$$

Resin beads are said herein to have a sulfonic acid group if the sulfonic acid group is bonded to a carbon atom of the polymer. The sulfonic acid group may be in the hydrogen form or in an anionic form with an associated cation.

Resin beads may also be classified according to the porosity of the beads, which is measured on dried resin using the Brunauer-Emmett-Teller (BET) method using nitrogen gas. Macroreticular ("MR") resin beads have number-average pore diameter of 50 nm to 500 nm. Gel resin beads have much smaller pores than MR resin beads. The number-average pore diameter of gel resin beads is often too small to measure properly with the BET method. The number-average pore diameter of gel resin beads is considered to be less than 20 nm.

Ratios are characterized herein as follows. For example, when a ratio is said to be 3:1 or greater, that ratio may be 3:1 or 5:1 or 100:1 but may not be 2:1. To state this in a general way, when a ratio is said herein to be X:1 or greater, it is meant that the ratio is Y:1, where Y is greater than or equal to X. Similarly, for example, when a ratio is said to be 15:1 or less, that ratio may be 15:1 or 10:1 or 0.1:1 but may not be 20:1. To state this in a general way, when a ratio is said herein to be W:1 or less, it is meant that the ratio is Z:1, where Z is less than or equal to W

The present invention is a composition that contains a collection of resin beads. Preferred resins contain polymerized units of one or more vinyl monomer. Preferably the amount of polymerized units of vinyl monomer in the resin is, by weight based on the weight of the resin, 95% or more; more preferably 99% or more.

Preferred resins contain polymerized units of one or more styrenic monomer. Preferred styrenic monomers are styrene, alkyl-substituted styrenes, divinylbenzene, and mixtures thereof; more preferred are styrene, divinylbenzene, and mixtures thereof. Preferably the amount of polymerized units of styrenic monomer in the resin is, by weight based on the weight of the resin, 50% or more; more preferably 75% or more; more preferably 85% or more; more preferably 95% or more; more preferably 99% or more.

Preferred resin beads are gel resin beads.

Preferably, the collection of resin beads has harmonic mean diameter of 100 μm or larger; more preferably 200 μm or larger; more preferably 400 μm or larger; more preferably 600 μm or larger. Preferably, the collection of resin beads has uniformity coefficient of 1.8 or smaller; more preferably 1.5 or smaller; 1.3 or smaller; more preferably 1.2 or smaller; more preferably 1.15 or smaller.

Preferably, the amount of polymerized units of multifunctional vinyl monomer is, by weight based on the weight of the resin, 0.5% or more; more preferably 1% or more; more preferably 1.5% or more. Preferably, the amount of polymerized units of multifunctional vinyl monomer is, by weight based on the weight of the resin, 15% or less; more preferably 10% or less; more preferably 8% or less; more preferably 6% or less. The preferred multifunctional vinyl monomer is divinylbenzene.

The resin may be characterized by the amount of sulfonic acid groups attached to the resin. Sulfonic acid groups are considered herein to contribute to this "amount" if they are attached to the resin and if the $SO_3$ group is intact. Thus, sulfonic acid groups contribute to this "amount" whether they are in hydrogenated form, in ionic form, ester form, in a complex with another chemical group, or some other form, as long as they are attached to the resin and the $SO_3$ groups are intact.

Preferably, the mole ratio of sulfonic acid groups attached to the resin to polymerized units of all monomers is 0.1:1 or greater; more preferably 0.2:1 or greater; more preferably 0.5:1 or greater; more preferably 0.75:1 or greater. Preferably, the mole ratio of sulfonic acid groups attached to the resin to polymerized units of all monomers is 2:1 or lower; more preferably 1.5:1 or lower; more preferably 1.3:1 or lower.

Preferably, the sulfonic acid groups are present in the resin at 0.3 mole per liter of resin (mol/L) or more; more preferably 0.4 mol/L or more; more preferably 0.5 mol/L or more; more preferably 0.6 mol/L or more. Preferably, the sulfonic acid groups are present in the resin at 4 mol/L or less; more preferably 3 mol/L or less; more preferably 2 mol/L or less.

The composition of the present invention contains one or more promoter. The promoter contains an amine group. The amine group is preferably selected from primary amines, secondary amines, tertiary amines, and heterocyclic aromatic groups in which a heteroatom in the cycle is a nitrogen; more preferably the amine group is selected from primary amines and heterocyclic aromatic groups in which a heteroatom in the cycle is a nitrogen; more preferably the amine group is a primary amine.

The promoter additionally contains a thiol group. Preferably the promoter contains no heteroatom other than the nitrogen atom in the amine group and the sulfur atom in the thiol group.

The promoters are selected from structures (III) and (IV)

$H_2N-CH_2-R^{15}-CH_2-SH$

(III)

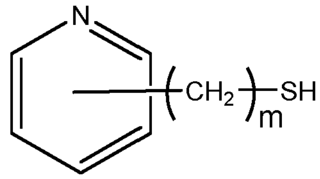

(IV)

where $R^{15}$ is $(CH_2)_p$ where p is 0 to 10, and m is 1 to 10; or 2,2-dimethylthiazolidine. Preferably, among structures (III), p is 0 to 5; more preferably p is 0 to 2; more preferably, p is 0. Preferably, among structures (IV), m is 1 to 3; more preferably 1. Among structures (IV), a preferred embodiment is 2-mercaptomethylpyridine. Structure (III) is preferred.

The promoter that has structure (III), where $R^{15}$ is absent, is cysteamine, also called 2-aminoethan-1-thiol. When 2,2-dimethylthiazolidine ("DMT") is added to resin beads, it is considered herein to be equivalent to adding cysteamine to the resin beads. This equivalence arises because water is normally present, and an equilibrium chemical reaction is established, with a mixture of DMT with water on one side of the chemical reaction and a mixture of cysteamine with acetone on the other side. Accordingly, the promoter may be 2,2-dimethylthiazolidine, cysteamine, or a mixture thereof.

Preferably, the amine group on the promoter molecule forms an ionic complex with the sulfonic acid group on the resin. That is, preferably, the hydrogen atom on an $SO_3H$ group on the resin partially or fully transfers to the amine group on the promoter, and the promoter remains in proximity to the sulfonic acid group.

Preferably, the mole ratio of promoter to sulfonic acid groups is 0.1:1 or more; more preferably 0.12:1 or more. Preferably, the mole ratio of promoter to sulfonic acid groups is 0.45:1 or less; more preferably 0.35:1 or less.

The composition of the present invention contains an antioxidant that has structure (I):

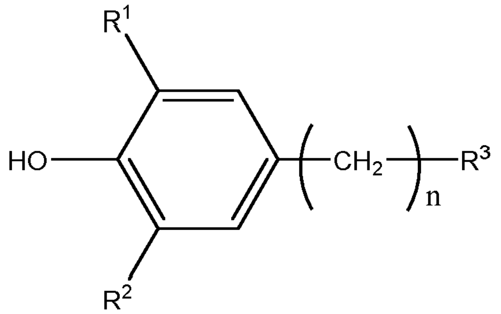

(I)

where each of $R^1$ and $R^2$ is an unsubstituted alkyl group containing 3 to 12 carbon atoms; wherein $R^3$ is hydrogen or methyl and n is 0 to 20.

Each of $R^1$ and $R^2$ contains 3 or more carbon atoms, more preferably 4 or more. Each of $R^1$ and $R^2$ contains 12 or fewer carbon atoms; more preferably 8 or fewer; more preferably 6 or fewer; more preferably 4 or fewer. Each of $R^1$ and $R^2$ is an unsubstituted alkyl. Preferably, each of $R^1$ and $R^2$ is t-butyl.

$R^3$ is hydrogen or methyl Preferably n is 15 or less; more preferably 10 or less; more preferably 5 or less; more preferably 3 or less; more preferably 1 or less; more preferably zero.

Preferably the amount of antioxidant is such that the mole ratio of antioxidant to sulfonic acid groups is 0.0002:1 or greater; more preferably 0.0005:1 or greater; more preferably 0.001:1 or greater. Preferably the amount of antioxidant is such that the mole ratio of antioxidant to sulfonic acid groups is 0.1:1 or lower; more preferably 0.05:1 or lower; more preferably 0.01:1 or lower.

The composition of the present invention may be made by any method. Preferably, the collection of resin beads is made by aqueous suspension polymerization of vinyl monomers, preferably including styrene and divinylbenzene. Preferably, after the polymerization is complete, the resin is subjected to one or more chemical reactions that attach sulfonic acid groups to the resin. Regardless of the method used for making the resin beads that have sulfonic acid groups, preferably the resin beads that have sulfonic acid groups are mixed with a promoter. Preferably the promoter forms an ionic complex with the sulfonic acid groups on the resin. Then, preferably, the collection of resin beads is washed with water, leaving some or all of the promoter attached to the resin beads, and the resin beads are preferably then dried, for example by heating to above 30°C under a pressure less than 1 atmosphere. The result of mixing promoter with the collection of resin beads is herein called "promoted resin beads."

Preferably, promoted resin beads are then mixed with antioxidant. When the antioxidant is not soluble in water,

the antioxidant may be dissolved in an organic solvent, acetone for example, to form a solution. The solution may be mixed with the promoted resin beads, and then the organic solvent may be evaporated.

While the present invention is not limited to any specific mechanism, it is contemplated that the degradation of antioxidants that were previously thought to be effective proceeds as follows. As an illustrative example, ETHANOX™ 703 (Albemarle Corporation), which has structure (V) below, may be considered. It has been discovered that ETHANOX™ 703 can degrade into the structure (VI) shown below. The carbon-carbon double bond external to the ring in structure (VI) can then react with a thiol group on a promoter to form structure (VII) or isomers thereof. The formation of structure (VII) ties up the thiol group and renders the promoter ineffective. The chemical reactions involved are shown schematically as follows, using cysteamine as an example of a promoter.

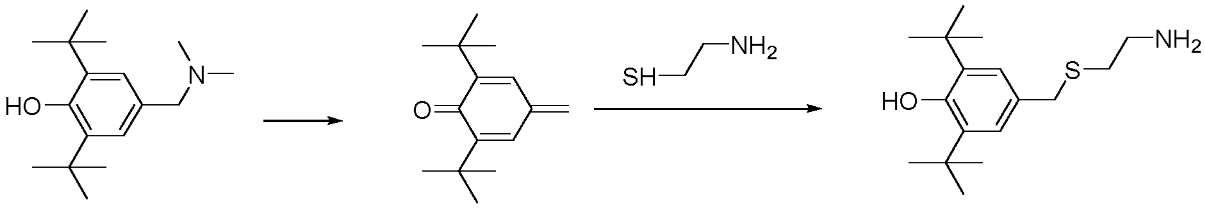

(V) (VI) (VII)

The following are examples of the present invention.

All operations were performed at room temperature (approximately 23°C) unless otherwise specified.

Example 1: LC/UV/MS testing of Solutions

Liquid Chromatograph with ultraviolet detector (LC/UV) conditions were as follows. ACCUCORE™ AQ C18 column (ThermoFisher Scientific) with a dimension of 150 x 4.6 mm, and a 5 μm particle; column temperature was held at 30 °C. The mobile phase A was water with 15 mM ammonium formate and 15 mM formic acid, and B was ACN with 0.1% formic acid. The following gradient was employed: 5% B (3 min) to 80% B within 15 min (hold for 5 min); flow rate was set at 1 mL/min. A diode array UV detector was used with scan range of 210 nm-600 nm, and the capacity to record data at various individual wavelengths. Injection volume was 1 uL for all samples.

Heated electrospray ionization (HESI) conditions were as follows: Spray voltage (+): 3500 V; Capillary Temperature: 320 °C; Sheath Gas: 35; Auxiliary gas: 10; Sweep gas: 3; Probe Heater Temperature: 110 °C; S-Lens RF Level: 50 V.

Mass spectrometry (MS) conditions were as follows. Full MS/dd-MS/MS mode. In-source CID: 5 eV; Default charge state: 2; Full MS resolution: 35,000; AGC target: 5e6; Maximum IT: 100 ms; Scan range: 70-1050 m/z; profile data type; dd-MS/MS resolution: 17,500; AGC target: 5e6, Maximum IT: 100 ms; Loop count: 3; MSX count: 1; TopN: 3; Isolation window: 4.0 m/z; NCE: 40 V, Stepped NCE: 50%.

Three antioxidants were used: ETHANOX™ 703 (comparative), as described above, BHT, and DtBP:

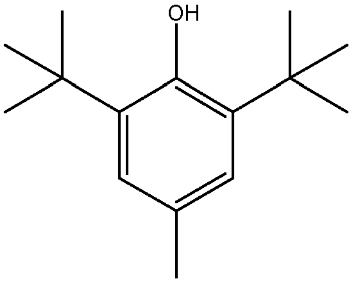

BHT

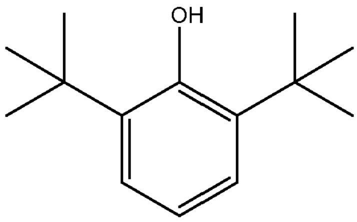

DtBP

The promoter used was 2,2-dimethylthiazolidine ("DMT"), which is known to convert to cysteamine when in contact with water.

Stock solutions were prepared in acetonitrile (ACN) as follows:

| Stock Solution | Solute | Concentration |
|---|---|---|
| SS-DMT | DMT | 7 mg/mL |
| SS-Eth | ETHANOX™ 703 | 8 mg/mL |
| SS-DtBP | DtBP | 10 mg/mL |
| SS-BHT | BHT | 10 mg/mL |

Test Mixtures were prepared as follows:

| Test Mixture | SS-DMT | SS-Eth | SS-DtBP | SS-BHT |
|---|---|---|---|---|
| Eth-only | | 100 μL | | |
| Eth/DMT | 100 μL | 100 μL | | |
| DtBP-only | | | 100 μL | |
| DtBP/DMT | 100 μL | | 100 μL | |
| BHT-only | | | | 100 μL |
| BHT/DMT | 100 μL | | | 100 μL |

Test mixtures were stored for 3 days at room temperature (approximately 23°C) and then diluted to 1 mL with ACN/water (50/50 by volume) for LC/UV/MS analysis.

The results for the Eth-only test mixture are shown in Fig. 1, which shows the relative intensity of the UV detector, using the 210-600 nm array. The peak at 14.3 minutes was identified by the MS analysis as the compound ETHANOX™ 703 (structure (V)).

The results for the Eth/DMT test mixture are shown in Fig. 2, which shows the relative intensity of the UV detector, using the UV detection array at 265-285 nm. The peaks below 5 minutes were shown by MS analysis to be degradation products of DMT, including cysteamine. The peak at 14.8 minutes was not present in the Eth-only test, and this peak was shown by MS analysis to be structure (VII). This demonstrates that, in the solution with both ETHANOX™ 703 and DMT, the two solutes react with each other. Further, the area of the peak at 14.3 minutes (ETHANOX™ 703) is smaller than the area of the corresponding peak in the Eth-only test. This verifies that the concentration of ETHANOX™ 703 is diminished in the presence of DMT.

Figures 3 and 4 compare DtBP-only with DtBP/DMT. The data shown correspond to a combination of a single wavelength in the UV detector of 275 nm. The peaks below 5 minutes were shown by MS analysis to be degradation products of DMT, including cysteamine. The peak at 18.6 minutes was identified by MS as DtBP itself. The peak areas for DtBP are the same in Figures 3 and 4, which shows that the concentration of DtBP did not decrease due to contact with DMT. Also, there are no peaks in Fig. 4 that corresponds to any reaction product between DtBP and DMT.

Figures 5 and 6 compare BHT-only with BHT/DMT. The data shown correspond to a single wavelength in the UV detector of 275 nm. The peaks below 5 minutes were shown by MS analysis to be degradation products of DMT, including cysteamine. The peak at 19.2 minutes was identified by MS as BHT itself. The peak areas for BHT are the same in Figures 5 and 6, which shows that the concentration of BHT did not decrease due to contact with DMT. Also, there are no peaks in Fig. 6 that corresponds to any reaction product between BHT and DMT.

Example 2: Mixture of Resin Beads, Promoter, and Antioxidant

The resin ("Resin-1") was AMBERLYST™ 131 (The Dow Chemical Company), used as supplied, in hydrogen form. Resin-1 is a gel resin with sulfonic acid groups, harmonic mean diameter of 700-800 μm, acid group concentration of ≥1.35 mol/L, and uniformity coefficient of less than 1.15.

The promoter was DMT. The antioxidants were ETHANOX™ 703 (comparative) and BHT.

A stock solution was prepared of 0.085 g ETHANOX™ 703 in 20 g of solution with acetone as solvent. A stock solution was prepared of 0.071 g BHT in 20 g of solution with acetone as solvent.

The following procedure was used.

Resin-1 was hot washed as follows. A vertical chromatography column equipped with circulated water jacket was used, with water circulating at 90°C. 120 mL of Resin-1 was loaded into the column. Then a fill/hold/drain cycle was performed: the column was filled with 1.2 bed volumes (BV) water and held for 40 minutes, and then the water was drained. Then the fill/hold/drain cycle was performed two more times. Then the resin was rinsed with 5 BV of deionized

water at 2 BV/hour.

Then Resin-1 was cold washed as follows. The hot water was drained from the recirculating jacket, and deionized water at approximately 23°C was flowed through the column at 4 BV/hr.

Then DMT was loaded onto the resin beads as follows. The sample of Resin-1 was placed into a 250 mL three-neck flask equipped with a stirrer, along with enough water to make a slurry. 4.75 g of DMT was dissolved into 30 mL of deionized water, which was then added dropwise into the slurry under stirring, and stirring continued for 1 hour. The stirring was stopped, and liquid was removed by siphon. The Resin-1 was transferred back to the column, and the resin was rinsed with 5 BV of deionized water approximately 23°C at 2 BV/hr.

Then antioxidant was loaded onto Resin-1 as follows. The resin was dried at 40°C in a vacuum oven. 20 g of the antioxidant stock solution (described above) was added, and the mixture stirred for 30 minutes. Then the acetone was removed by evaporation in a vacuum oven at approximately 23°C.

The Resin-1, loaded with promoter and antioxidant as described above, was then put into a bottle, kept in an oven at 80°C for 2 hours, then stored at approximately 23°C for 7 days.

50 g of Resin-1 and 150 g of deionized water were shaken in a container for 20 minutes at approximately 23°C. Using a gravity funnel and filter paper, the bulk liquid was separated from the resin beads. The conductivity and the pH of the bulk liquid was measured using an Expanded Range Conductivity Meter #23226-523 (VWR International) and a pH meter.

It is considered that the exposure to 80°C for two hours provides a test of the stability of the catalyst resin when exposed to long term and/or elevated-temperature storage.

Results were as follows:

| Antioxidant | pH | Conductivity |
|---|---|---|
| ETHANOX™ 703 (Comparative) | 3.17 | 281.5 μS |
| BHT | 3.23 | 241.5 μS |

When a resin loaded with promoter and antioxidant is stored, it is normally observed that, as the resin, the promoter, and/or the antioxidant degrade, the pH falls and the conductivity rises. The above table shows that BHT preserves the composition, because the BHT sample had higher pH and lower conductivity.

## Claims

**1.** A catalyst composition comprising

(a) a collection of resin beads having sulfonic acid functional groups;
(b) one or more promoter selected from structures (III) and (IV)

$$H_2N-CH_2-R^{15}-CH_2-SH$$

(III)

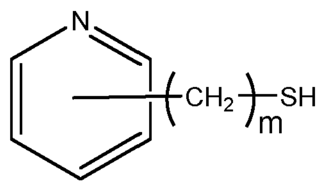

(IV)

where $R^{15}$ is $(CH_2)_p$ where p is 0 to 10, and m is 1 to 10; or 2,2-dimethylthiazolidine; and
(c) an antioxidant having the structure (I)

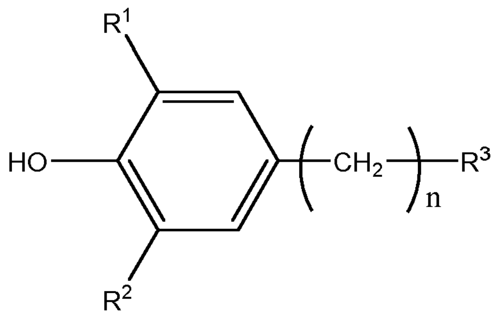

(I)

wherein each of $R^1$ and $R^2$ is an unsubstituted alkyl group containing 3 to 12 carbon atoms;
wherein $R^3$ is hydrogen or methyl and n is 0 to 20.

**2.** The catalyst composition of claim 1, wherein each of $R^1$ and $R^2$ is t-butyl.

**3.** The catalyst composition of claim 1, wherein $R^3$ is hydrogen, and n is zero.

**4.** The catalyst composition of claim 1, wherein $R^3$ is methyl, and n is zero.

**5.** The catalyst composition of claim 1, wherein the resin comprises polymerized units of one or more styrenic monomers.

**6.** The catalyst composition of claim 1, wherein the promoter is 2,2-dimethylthiazolidine, cysteamine, or a mixture thereof.

**Patentansprüche**

**1.** Katalysatorzusammensetzung umfassend

(a) eine Ansammlung von Harzperlen, die sulfonsäurefunktionelle Gruppen aufweisen;
(b) einen oder mehrere Promotoren ausgewählt unter den Strukturen (III) und (IV)

$H_2N-CH_2-R^{15}-CH_2-SH$

(III)

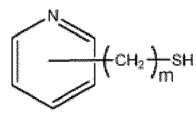

(IV)

wobei $R^{15}$ $(CH_2)_p$ ist, wobei p 0 bis 10 beträgt und m 1 bis 10 beträgt, oder 2,2-Dimethylthiazolidin ist; und
(c) ein Antioxidationsmittel, das die Struktur (I)

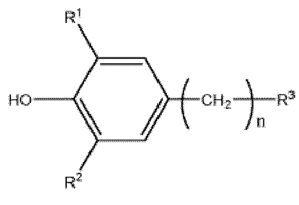

(I)

aufweist, wobei jedes von $R^1$ und $R^2$ eine unsubstituierte Alkylgruppe ist, die 3 bis 12 Kohlenstoffatome enthält; wobei $R^3$ Wasserstoff oder Methyl ist und n 0 bis 20 beträgt.

**2.** Katalysatorzusammensetzung nach Anspruch 1, wobei jedes von $R^1$ und $R^2$ t-Butyl ist.

**3.** Katalysatorzusammensetzung nach Anspruch 1, wobei $R^3$ Wasserstoff ist und n null beträgt.

4. Katalysatorzusammensetzung nach Anspruch 1, wobei $R^3$ Methyl ist und n null beträgt.

5. Katalysatorzusammensetzung nach Anspruch 1, wobei das Harz polymerisierte Einheiten eines oder mehrerer styrolischer Monomere umfasst.

6. Katalysatorzusammensetzung nach Anspruch 1, wobei der Promotor 2,2-Dimethylthiazolidin, Cysteamin oder eine Mischung davon ist.

## Revendications

1. Composition catalyseur comprenant

(a) une collection de billes de résine ayant des groupes fonctionnels acide sulfonique;
(b) un ou plusieurs promoteurs sélectionnés parmi les structures (III) et (IV)

$H_2N-CH_2-R^{15}-CH_2-SH$

(III)

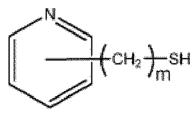

(IV)

où $R^{15}$ est $(CH_2)_p$ où p a la valeur de 0 à 10, et m a la valeur de 1 à 10; ou est la 2,2-diméthylthiazolidine; et
(c) un agent antioxydant ayant la structure (I)

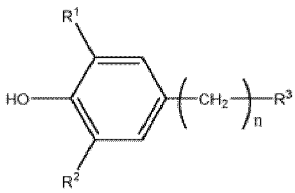

(I)

chacun de $R^1$ et $R^2$ étant un groupe alkyle non substitué contenant de 3 à 12 atomes de carbone;
$R^3$ étant un hydrogène ou un méthyle et n ayant la valeur de 0 à 20.

2. Composition catalyseur selon la revendication 1, chacun de $R^1$ et $R^2$ étant un groupe *t*-butyle.

3. Composition catalyseur selon la revendication 1, $R^3$ étant un hydrogène, et n ayant la valeur de zéro.

4. Composition catalyseur selon la revendication 1, $R^3$ étant un méthyle, et n ayant la valeur de zéro.

5. Composition catalyseur selon la revendication 1, la résine comprenant des motifs polymérisés d'un ou de plusieurs monomères styréniques.

6. Composition catalyseur selon la revendication 1, le promoteur étant la 2,2-diméthylthiazolidine, la cystéamine, ou un mélange de celles-ci.

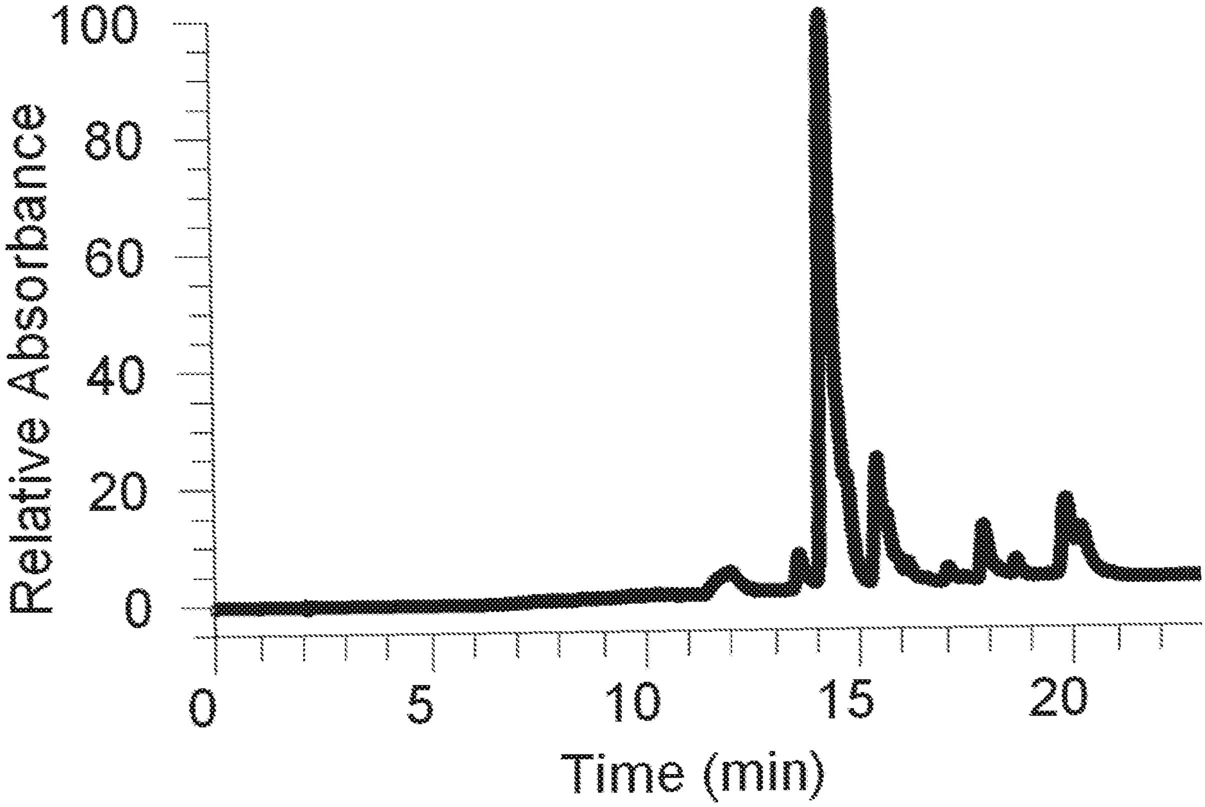


Fig. 1

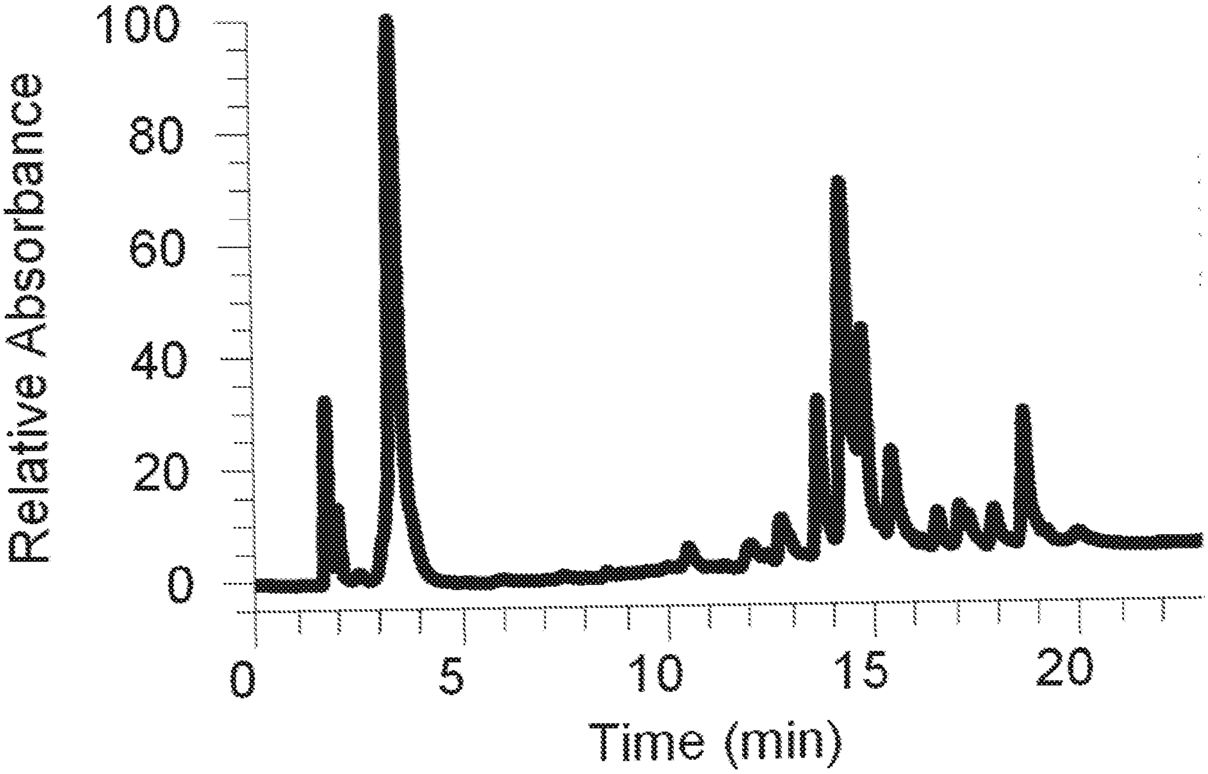


Fig. 2

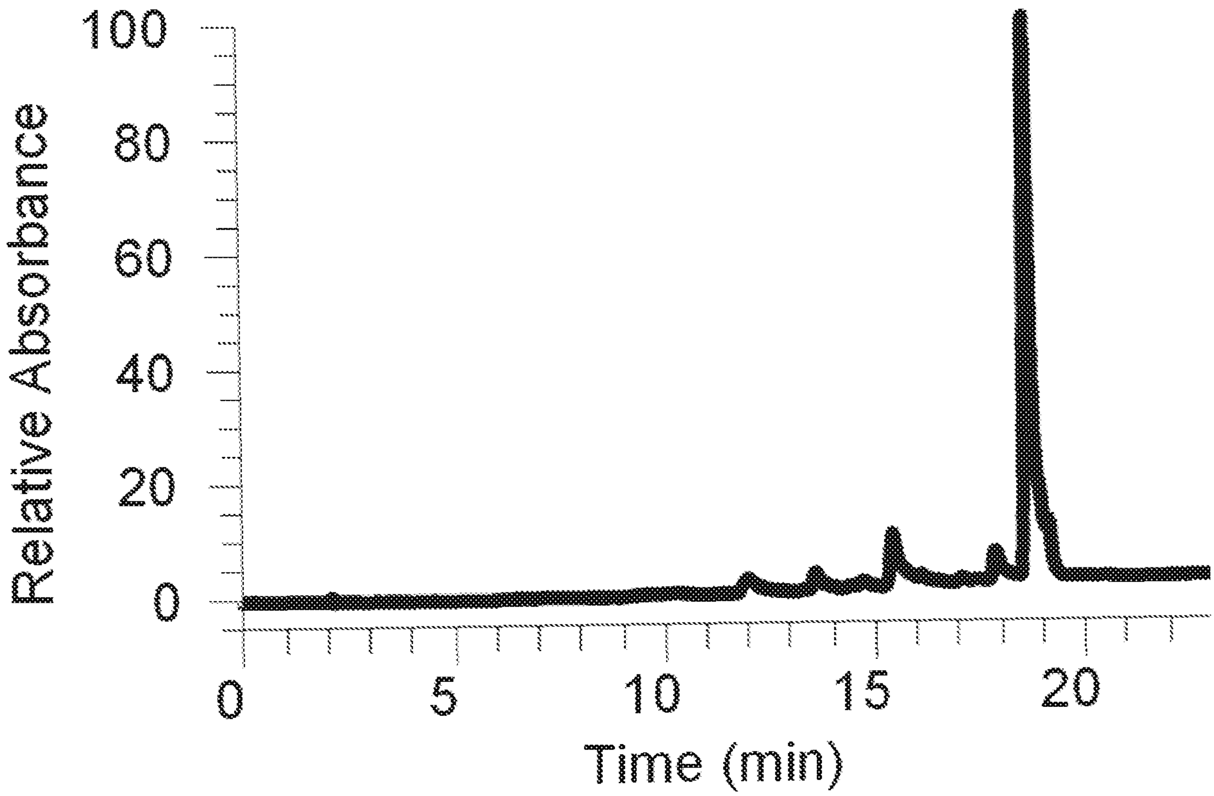


Fig. 3

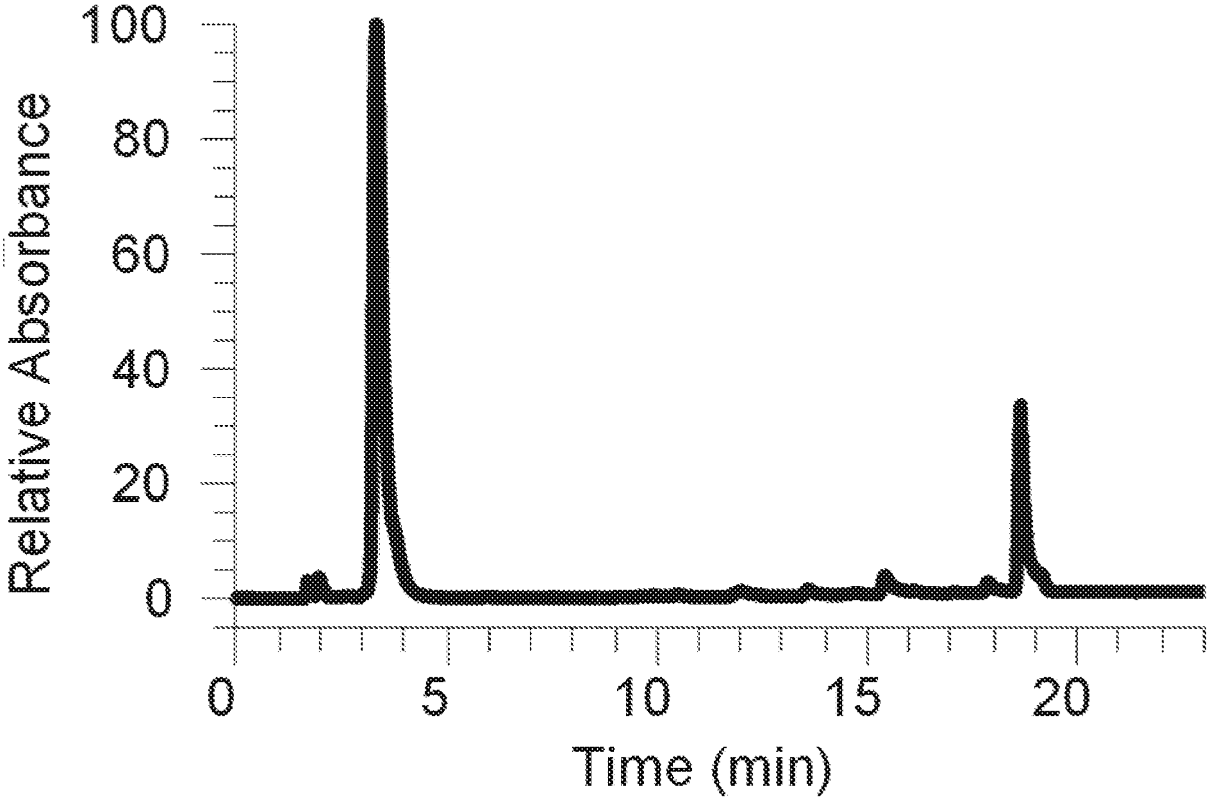


Fig. 4

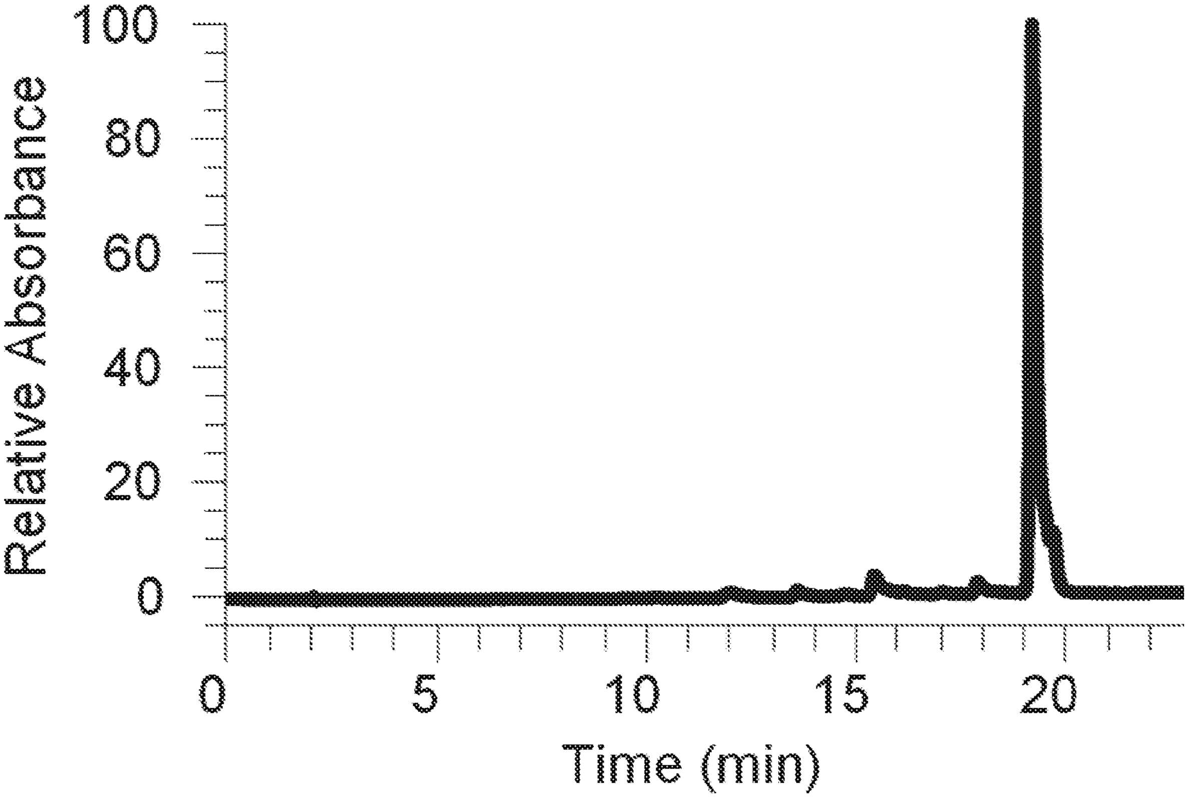


Fig. 5

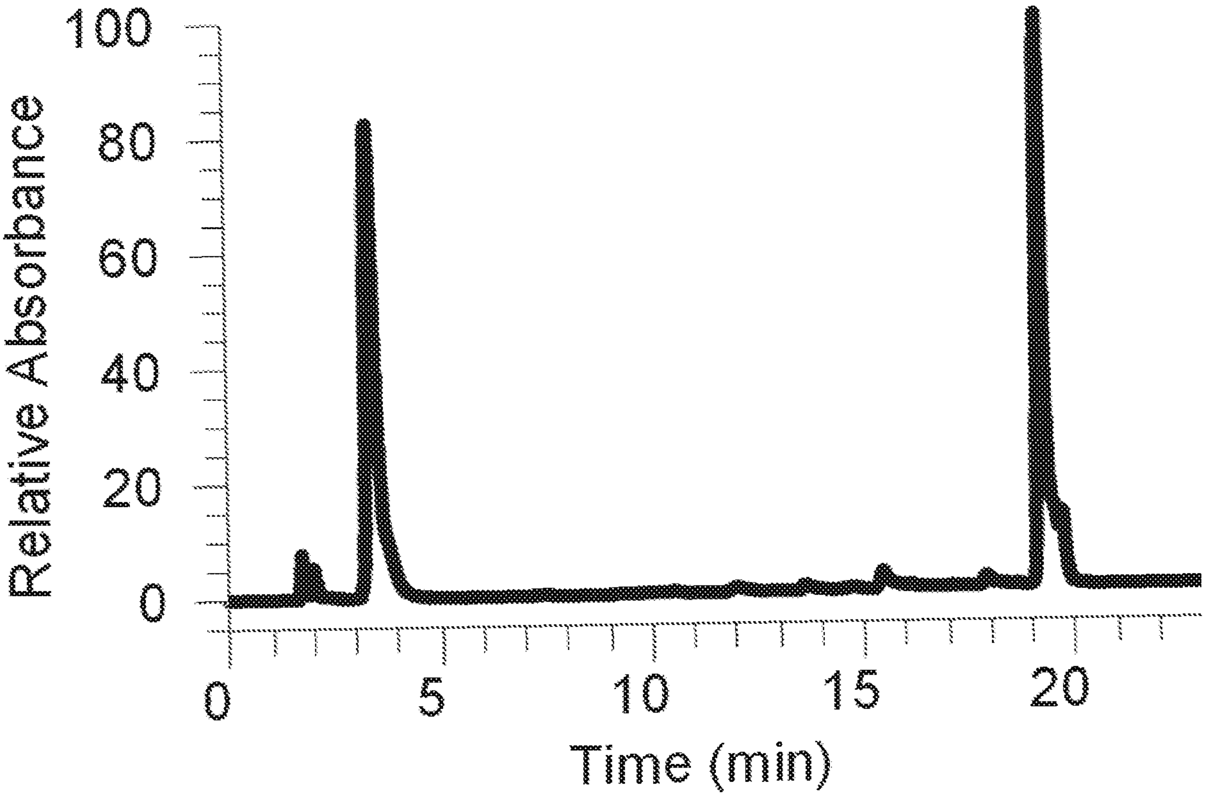


Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8907138 B
- US 20140378715 A